# EUROPEAN PATENT APPLICATION

(11) **EP 1 923 051 A1**
(43) Date of publication of application: **21.05.2008**
(21) Application number: 06783174.3
(22) Date of filing: 05.09.2006
(51) Int. Cl.: A61K 9/14, A61K 47/02, A61K 47/12, A61K 47/26, A61K 47/36, A61K 47/38

(54) **MICROPARTICLE OF HARDLY-SOLUBLE SUBSTANCE HAVING ENTERIC BASE MATERIAL ADSORBED ON THE SURFACE OF THE SUBSTANCE**

(30) Priority: 06.09.2005 US 714924 P
(71) Applicant: Astellas Pharma Inc., Tokyo 103-8411 (JP)
(72) Inventor: YAMAGUCHI, Hisami, Astellas Pharma Inc., Tokyo 1038411 (JP); TOMINAGA, Tetsuo, Astellas Pharma Inc., Tokyo 1038411 (JP)
(74) Representative: Bates, Philip Ian
(86) International application number: PCT/JP2006/317483
(87) International publication number: WO 2007/029660

(57) **Abstract**

The present invention relates to fine particles of a poorly soluble drug to provide pharmaceutical preparation having an improved absorption by improving dissolution profile of a poorly soluble drug having a poor absorbability, as well as a method for producing the same. In particular, the present invention relates to fine particles of a poorly soluble drug, having an average particle diameter of 1 nm to 1,000 nm, wherein a particular type of an enteric base material is adsorbed on the surface of the poorly soluble drug, fine particles which further comprises a saccharide, as well as a method for producing the same. It is possible to efficiently and safely produce in a short amount of time fine particles with which absorption of a poorly soluble drug that is poorly absorbed in humans, and the like can be improved, and a pharmaceutical preparation with excellent dispersion stability can be provided, by using the fine particles of the present invention having an improved dissolution profile.

## Description

### Technical Field

The present invention pertains to the fine particles of a poorly a, poorly soluble drug that are necessary for providing a pharmaceutical preparation of improved absorption by improving the dissolution of the poorly soluble drug, and a method for producing the same fine particles. In further detail, the present invention relates to fine particles of a poorly soluble drug with an average particle diameter of 1 to 1,000 nm wherein a predetermined enteric base material has been adsorbed on the surface of a poorly soluble drug; fine particles further containing a sugar; and a method for producing the same.

### Background Art

There are many times when the quality of oral absorption of a drug active ingredient is the key to development of a pharmaceutical drug. It is preferred that a candidate for development as an oral drug have excellent solubility because the drug effect of virtually any oral drug is manifested as a result of the active ingredient being dissolved and absorbed in the digestive tract. Nevertheless, there are also reports that today 30 to 40% of the candidates for development as a pharmaceutical drug do not attain the necessary solubility for an oral agent (Am. Pharm. Rev. 5, p. 82-85 (2002)). Therefore, technology for improving the dissolution of a poorly soluble drug is very important technology that is fundamental to the development of drugs. Methods whereby a drug substance is dry pulverized by a pin mill or jet mill and methods whereby a drug substance is dissolved in an organic solvent to make a soft capsule have often been used in the past as methods for improving dissolution. Nevertheless, there are many cases in which the average particle diameter after pulverization by jet mill or other dry pulverization methods is on the order of several microns at best and sufficient results in terms of improving dissolution cannot be expected.
In addition, there is a problem with soft capsules in that the maximum size of a capsule that can be administered is 2 mL, and only the dose of drug that can be dissolved in this amount of organic solvent can therefore be given.

In order to solve the above-mentioned problems, a method whereby a drug is made into a solid dispersion was developed as a technology with which solubility is sufficiently improved and it is guaranteed that a high dose will be administered. By means of this method, the active ingredient is dispersed and solidified in amorphous state in polyvinylpyrrolidone (abbreviated PVP hereafter), hydroxypropylmethyl cellulose (abbreviated HPMC hereafter), or another soluble polymer vehicle, and solubility up to super-saturation can be temporarily improved. A method whereby a poorly soluble drug and a soluble polymer are melted at a high temperature and a method whereby these starting materials are dissolved in an organic solvent and then dried are known production methods. The latter method whereby an organic solvent is employed have been widely set to practical use.

Nevertheless, there are three problems with this type of solid dispersion technology for industrial production. The first is a problem relating to production. Ethanol, dichloromethane, or another organic solvent must be used in order to dissolve the poorly soluble drug and support the drug in a polymer vehicle. This is undesirable in terms of guaranteeing safety during the manufacturing process and protecting the environment, and there is a chance that organic solvent will remain in the solid dispersion after production. Moreover, there is also a problem with melting at high temperatures in that drugs that are unstable at high temperatures cannot be used. The second problem is that there are often cases in which a large amount of polymer vehicle is needed, with it becoming necessary to add a vehicle in an amount that is at least five-times the amount of drug substance in order to guarantee sufficient supersaturated solubility. When such large amounts of vehicle must be added, there is an extreme increase in the size of the tablets or capsules and this compromises patient compliance; therefore, development of the drug must in essence be abandoned. Furthermore, a third problem relates to product stability. When a solid dispersion produced in this manner is stored under high humidity, the drug molecules that exist in an amorphous state proceed to recrystallize, there is a reduction in supersaturated solubility, and the drug dissolution profile deteriorates as a result.

Methods whereby a suspension of drug particles with an average particle diameter of 400 nm or smaller is prepared are considered to be promising methods that effect a breakthrough in this situation (for instance, refer to Patent Reference 1). These are methods whereby the particle surface area is dramatically increased by subjecting a poorly soluble drug and PVP or another dispersant to wet pulverization using a bead mill in order to improve the dissolution profile of a poorly soluble drug. By means of such methods, it is necessary to add a dispersant as an essential ingredient for producing fine particles, but there is also an advantage in that the amount of dispersant added promises to be less than the amount of polymer vehicle that is needed by the solid dispersion method and the drug can be produced without using an organic solvent. Moreover, this technology has recently received attention as a technology that is superior in the sense that stability is guaranteed without the reduction in supersaturated solubility and deterioration of the dissolution profile during storage under high humidity that is seen with solid dispersions because it is not a technology that targets supersaturated solubility of a drug.
[Patent Reference 1] Specification of USP 5,145,684

### Disclosure of the Invention

### Problem to be solved

In light of this background, the inventors focused on methods for producing fine particles by wet pulverization using a bead mill, high-pressure emulsifier, rotary disk mill, and the like and concluded as a result of evaluating the utility of such a method that there are several problem points.

One problem is that the pulverization time is generally very long, and when a bead mill is used as the pulverization mill, it must often continue to work for five to eight days on a production scale. Therefore, there is an obvious drop in production efficiency and rise in cost. The long pulverization time increases the chance that equipment problems will be encountered during that time, and is also undesirable in terms of production management by GMP. When there are problems during wet pulverization, it is extremely difficult to efficiently recover the intermediate product suspension during production and avoid contamination by microorganisms, and the like. In addition, bead mill methods in particular pulverize by mechanical force; therefore, there is a problem that cannot be disregarded in that abrasion of the inside walls of the container, beads, and the like is inevitable and there is an increase in the amount of impurities that mix in the suspension when the equipment is operated for long periods of time.
Thus, tests to improve pulverization efficiency by wet pulverization are being conducted primarily by equipment manufacturers, and new models such as the ECM Dynomill made by WAB (Switzerland) with a unique bead mill rotor shape and the UVM-2 Ultraviscomll made by Aimex Corporation (Japan) are now on the market. Moreover, many attempts have been made to change the very structure and mechanism of the equipment, and wet pulverization mills characterized by excellent pulverization efficiency are now being sold by different countries throughout the world. Methods that use high-pressure emulsifiers have also been proposed. Nevertheless, machines that operate with stability and are capable of producing fine particles with an average particle diameter of 50 to 1,000 nm have not been successful in sufficiently curtailing the pulverization time.

Another problem is the dispersion stability of a fine particle suspension. When stability during storage and during dilution of a fine particle suspension obtained using a high-pressure emulsifier or bead mill were evaluated, it was concluded that there was a tendency toward particles aggregating over time, and that when the drug is a basic, poorly soluble drug that tends to dissolve in the acidic region, occasionally this aggregation tendency is strong and sedimentation takes places. It was also concluded that conventional fine particle suspensions aggregate very easily in electrolyte solutions, and it appeared that there could be a problem with safety when these suspensions are administered as injections.
Solutions to the above-mentioned problems relating to production and aggregation of particles were felt to be important to the use of fine particle suspension technology on an industrial basis in order to improve absorption of a poorly soluble drug.

### Means of solving problems

The inventors focused on dispersants that are added during nanosuspension production and intensely studied the effects of various pharmaceutical additives as dispersants in order to solve the problems associated with production and dispersion of fine particle suspensions. As a result, they discovered that these problems are solved by dissolving hydroxypropylmethyl cellulose phthalate (abbreviated as HPMCP hereafter) or hydroxypropylmethyl cellulose acetate succinate (abbreviated as HPMCAS hereafter), which had been rejected because of their poor solubility in water, in an aqueous solution of an alkali such as sodium citrate, and using this solution as a dispersant.

Fine particles in a suspension apparently display excellent dispersion stability due to the repulsive force of the zeta potential at the particle surface. It is known that when the zeta potential at the fine particle surface is neutralized, there is a tendency toward aggregation of particles. It was believed that dissolving HPMCAS or HPMCP in an electrolyte solution and using that solution as a dispersant would not be advantageous in terms of producing a suspension with excellent dispersion stability (for instance, G. W. Castellan, Physical Chemistry, Third Edition, Section 18.16.3; translation, Meguro, Tanaka, Imamura, translators: G.W. Castellan, Physical Chemistry (1), Third Edition, p. 474, Tokyo Kagaku Dojin (1986) and C. Keck et al.: Production and optimization of oral cyclosporine nanocrystals, Abstract of AAPS Annual Meeting (2004)). Nevertheless, the inventors discovered that when, unfettered by conventional wisdom relating to particle aggregation, HPMCAS or HPMCP was dissolved in an alkali solution in which a large amount of sodium citrate had been dissolved and a poorly soluble drug was pulverized in this solution, fine pulverization proceeded more quickly than when conventional HPMC or PVP was used, and contrary to expectations, dispersibility of the drug fine particles was very good.

Moreover, it is necessary to add a dispersant that dissolves easily in purified water and to finely pulverize the poorly soluble drug in this solution when producing a suspension of fine particles of a drug by wet pulverization. Of the various dispersants that are known, PVP or Pluronic F68 and F108 have been particularly preferred because they are freely soluble in water and do not require an alkali or other electrolyte as a resolvent.
The inventors studied dispersants not previously cited as examples, focusing on the effect of these dispersants on productivity and dispersion stability in order to improve productivity by fine pulverization in a short amount of time and improve dispersion stability of a suspension when a suspension of fine particles of a drug is produced by wet pulverization. As a result, they discovered that when, unfettered by conventional wisdom relating to particle aggregation, HPMCAS and/or HPMCP is dissolved in water by addition of an alkali and this solution is used as the dispersant for fine pulverization of a drug, pulverization surprisingly proceeds very efficiently and a suspension with excellent dispersion stability is obtained: Moreover, when the suspension produced in this way is dried, the product releases fine particles as the HMPCAS or HPMCP dissolves in fluid No. 2 of the Japanese Pharmacopoeia Dissolution test (pH of 6.8). The inventors successfully completed the present invention upon discovering that when a sugar and/or a sugar alcohol is added to this suspension and the product is dried, the product has particularly excellent properties in terms of redispersion without aggregation of the fine particles.

That is, the present invention relates to:
1. fine particles of a poorly soluble drug, having an average particle diameter of 1 nm to 1,000 nm, wherein hydroxypropylmethyl cellulose acetate succinate and/or hydroxypropylmethyl cellulose phthalate are adsorbed on the surface of the poorly soluble drug;
2. the fine particles according to the above-mentioned 1, which comprise hydroxypropylmethyl cellulose acetate succinate and/or hydroxypropylmethyl cellulose phthalate at a ratio of 0.005 to 20 parts by weight per part by weight of the poorly soluble drug;
3. the fine particles according to either of the above-mentioned 1 and 2, which further comprise 0.01 to 4,000 parts by weight of a sugar and/or a sugar alcohol per part by weight of the poorly soluble drug;
4. the fine particles according to the above-mentioned 3, wherein the sugar and/or the sugar alcohol is one or more selected from the group consisting of lactose, fructose, sucrose, glucose, oligosaccharides, mannitol, sorbitol, maltitol, maltose, xylitol, erythritol, reduced thick maltose syrup, trehalose, anhydrous lactose, and xylose;
5. the fine particles according to any of the above-mentioned 1 through 4, which are obtainable by a production method selected from the following (1) through (3):
   (1) a production method characterized in that a poorly soluble drug is dispersed in a solvent in which hydroxypropylmethyl cellulose acetate succinate and/or hydroxypropylmethyl cellulose phthalate and a resolvent therefor have been dissolved or suspended, and then the average particle diameter of the poorly soluble drug is reduced by further subjecting the resulting mixture to wet pulverization,
   (2) a production method characterized in that an organic solvent solution in which a poorly soluble drug has been dissolved is added to a solution in which hydroxypropylmethyl cellulose acetate succinate and/or hydroxypropylmethyl cellulose phthalate and a resolvent therefor have been dissolved or suspended and fine particles of a poorly soluble drug are precipitated, or
   (3) a production method characterized in that a solvent in which hydroxypropylmethyl cellulose acetate succinate and/or hydroxypropylmethyl cellulose phthalate and a resolvent therefor have been dissolved or suspended is added to the pulverized product resulting from wet pulverization of a poorly soluble drug in the presence of a dispersant;
6. the fine particles according to the above-mentioned 5, wherein the resolvent for hydroxypropylmethyl cellulose acetate succinate and/or hydroxypropylmethyl cellulose phthalate is an alkaline substance or a substance that electrolytically dissociates alklali metal ions or alkali earth metal ions in water;
7. the fine particles according to the above-mentioned 5 and 6, wherein the alkaline substance or the substance that electrolytically dissociates alkali metal ions or alkali earth metal ions in water is one or more substances selected from the group consisting of sodium citrate, calcium citrate, citrates, sodium tartrate, sodium malate, sodium lactate, sodium hydroxide, potassium hydroxide, calcium hydroxide, magnesium hydroxide, sodium carbonate, sodium bicarbonate, triethanolamine, monoethanolamine, magnesium aluminum silicate, phosphate, magnesium oxide, calcium oxide, *L*-arginine, aqueous ammonia, and sodium alginate;
8. a method for producing fine particles of poorly soluble drug, having an average particle diameter of 1 nm to 1,000 nm, wherein hydroxypropylmethyl cellulose acetate succinate and/or hydroxypropylmethyl cellulose phthalate are adsorbed on the surface of the poorly soluble drug, characterized in that hydroxypropylmethyl cellulose acetate succinate and/or hydroxypropylmethyl cellulose phthalate and a resolvent therefor are dissolved or suspended in water or a mixture of a water-soluble organic solvent and water, a poorly soluble drug is dispersed therein, and then the average particle diameter of the poorly soluble drug is reduced by further subjecting the resulting mixture to wet pulverization;
9. a method for producing fine particles of poorly soluble drug, having an average particle diameter of 1 nm to 1,000 nm, wherein hydroxypropylmethyl cellulose acetate succinate and/or hydroxypropylmethylcellulose phthalate are adsorbed on the surface of the poorly soluble drug, characterized in that an organic solvent solution in which the poorly soluble drug has been dissolved is added to a solution in which hydroxypropylmethyl cellulose acetate succinate and/or hydroxypropylmethyl cellulose phthalate and a resolvent therefor have been dissolved or suspended, and fine particles of poorly soluble drug are precipitated; and
10. a method for fine particles of poorly soluble drug, having an average particle diameter of 1 nm to 1,000 nm, wherein hydroxypropylmethyl cellulose acetate succinate and/or hydroxypropylmethyl cellulose phthalate are adsorbed on the surface of the poorly soluble drug, characterized in that a solvent in which hydroxypropylmethyl cellulose acetate succinate and/or hydroxypropylmethyl cellulose phthalate and a resolvent therefor have been dissolved or suspended is added to the pulverized product obtainable by wet pulverization of a poorly soluble drug in the presence of a dispersant.

The present invention will now be described in detail.
There are no special restrictions to the poorly soluble drug used in the present invention as long as it is poorly soluble in water, but the phrase "poorly soluble drug" specifically means a drug having a solubility in purified water of 0.1 mg/mL or less, preferably 0.05 mg/mL or less, when solubility is evaluated by preparing the free form or a hydrate of the free form. The drug can be selected from a variety of known drugs, including analgesics, anti-inflammatory drugs, anthelmintics, anti-arrhythmic agents, antibiotics (including penicillins), anticoagulants, antihypertensives, antidiabetic agents, antiepileptics, antihistamines, anti-hypertensive drugs, antimuscarinic agents, antimycobacterial agents, antineoplastic agents, immunosuppressants, antithyroid drugs, antiviral agents, anxiolytic drugs (sleep aids and nerve relaxants), astringents, β adrenoreceptor blocking agents, blood preparations and plasma substitutes, drugs used for myocardial degeneration, contrast media, corticosteroids, cough suppressants (expectorants and mucolytics), diagnostic drugs, diagnostic imaging drugs, diuretics, dopaminergic drugs (anti-Parkinson's drugs), hemostatics, immunological drugs, lipid regulating agents, muscle relaxants, parasympathomimetics, parathyroid calcitonin and biphosphonates, prostaglandins, radiopharmaceuticals, sex hormones (including steroids), anti-allergic agents, stimulants and appetite suppressants, sympathomimetic drugs, thyroid agents, vasodilators, and xanthines. Specific examples are nifedipine, tacrolimus, indomethacin, diclofenac sodium, nifedipine, aspirin, ibuprofen, naproxen, furosemid, oxolinic acid, warfarin potassium, FK555 (ASP0355), dicoumarol, phenytoin, phenobarbital, ketoprofen, chlorpropamide, griseofulvin, carbamazepin, cyclosporine A, dermazole, ketoconazole, prednisone, triamsinalone acetonide, bromoureryl urea, acetyl tilosine, vinpocetine, domperidone, allopurinol, tolipamide, indapamide, oxatomide, hepronicate, pindolole.
The effect of the present invention in terms of improving production and aggregation of the fine particle suspension of the present invention is much more obvious with basic, poorly soluble drugs that have proven particularly difficult when using prior art.
There are no special restrictions to the basic, poorly soluble drug of the present invention as long as it is poorly soluble in water and is basic, but the phrase "basic, poorly soluble drug" refers to a drug having a solubility in purified water of 0.1 mg/mL or less and a solubility at a pH of 1.2 that is at least twice the solubility in purified water, preferably a solubility in purified water of 0.05 mg/mL or less and a solubility at a pH of 1.2 that is at least three times the solubility in purified water. Examples are FK4664, guanfacine hydrochloride, manidipine hydrochloride, tamoxifen citrate, and nicardipine hydrochloride.

The ratio of the poorly soluble drug contained in the fine particles of the present invention in terms of the total of fine particles should be 0.1 to 99.9 wt%, preferably 0.5 to 99 wt%, particularly 10 to 95 wt%, ideally 20 to 90 wt%.
There are no special restrictions to the degree of substitution of the HPMCAS that is used as dispersant in the present invention, but a methoxyl group content of 10 to 29%, hydroxypropoxyl group content of 2 to 25%, acetyl group content of 1 to 18%, and succinoyl group content of 2 to 30% are preferred, and a methoxyl group content of 19 to 27%, hydroxypropoxyl group content of 4 to 11 %, acetyl group content of 4 to 15%, and succinoyl group content of 3 to 19% are particularly preferred. The AS-LG, AS-LF, AS-MG, AS-MF, AS-HG, and AS-HF grades supplied as AQOAT by Shin-Etsu Chemical Co., Ltd. are ideal. Moreover, there are no special restrictions to the degree of substitution of the HPMCP that is used as a dispersant in the present invention, but a methoxyl group content of 16 to 27%, hydroxypropoxy group content of 3 to 12%, and a carboxybenzoyl group content of 19 to 37% are preferred, and a methoxyl group content of 18 to 24%, hydroxypropoxyl group content of 5 to 10%, and carboxylbenzoyl group content of 21 to 35% are particularly preferred. The HP-50, HP-55, and HP-55S supplied by Shin-Etsu Chemical Co., Ltd. are ideal. Moreover, the amount of HPMCP or HPMCAS added per part by weight of poorly soluble drug in the present invention is 0.005 to 20 parts by weight, preferably 0.02 to 10 parts by weight, particularly 0.05 to 5 parts by weight, of HPMCP or HPMCAS. HPMCAS is preferred over HPMCP as the dispersant of the present invention.

The dispersant used together with HPMCP and/or HPMCAS in the present invention is one or more substances selected from hydroxypropylmethyl cellulose, hydroxypropyl cellulose, hydroxyethyl cellulose, hydroxyethylmethyl cellulose, methyl cellulose, and other soluble celluloses, ethyl acrylate-methyl methacrylate copolymer, methacrylic acid copolymer, aminoalkyl methacrylate copolymer, gum arabic, sodium alginate, pregelatinized starch, reduced thick maltose syrup, casein sodium, dextrin, tragacanth powder, pullulan, propyl glycol, pectin, sodium polyacrylate, lecithin, polyvinyl alcohol, polyethylene glycol, thick maltose syrup, polyvinyl pyrrolidone, and polyoxyethylene poloxypropylene glycol, Pluronic F68, Pluronic F108, polysorbate 80, polysorbate, sodium laurylsulfate, polyoxyl stearate 40, poloxyethylene-cured castor oil, sorbitan fatty acid ester, sucrose fatty acid ester, polyoxyethylene alkyl ether, polyoxyethylene alkyl phenyl ether, sodium sulfosuccinate, dioctyl sodium sulfosuccinate, and other surfactants.

The state where a dispersant is adsorbed on the surface of the poorly soluble drug of the fine particles of the present invention means a state wherein the HPMCP, HPMCAS or other dispersant is chemically adsorbed by bonding chemically with the surface or is physically adsorbed.

The fine particles of the present invention should be a particulate composition with an average particle diameter of 1 nm to 1,000 nm, and the average particle diameter is preferably 1 to 750 nm, particularly 1 to 500 nm, ideally 1 to 300 nm. This average particle diameter means the median diameter by volume criterion, and it can be measured by conventional methods for measuring particle size in the technical field in question, for instance, methods for measuring particle diameter by laser scattering, precipitation field flow fractionation, photon correlation spectroscopy, or disk centrifugation, but it is preferred that the average particle diameter be measured using the HORIBA LA-920 (Horiba Ltd.) for measuring particle diameter by laser scattering.
In addition, improved dispersibility is cited as a characteristic of the fine particles of the present invention. Specifically, the particles are characterized in that a suspension containing the fine particles of the present invention will not display any macroscopic aggregation or precipitation six months or longer after preparation, preferably the average particle diameter of a suspension containing the fine particles of the present invention will not increase after preparation, and for at least three months any increase in size will be kept to less than twice the original particle diameter.

Examples of the sugar and sugar alcohol of the present invention are lactose, fructose, sucrose, glucose, erythritol, xylitol, mannitol, trehalose, lactose anhydride, sorbitol, maltitol, arabinose, xylose, fructose, galactose, mannose, lactitol, xylose, maltose, maltotriose, panose, lactosucrose, teandalose, reduced lactose, and one or a combination of two or more of these can be used in the present invention. Moreover, the amount of sugar and sugar alcohol added per part by weight of the drug is 0.01 to 4,000 parts by weight, preferably 0.01 to 400 parts by weight, particularly 0.02 to 200 parts by weight, ideally 0.05 to 200 parts by weight.

The phrase "resolvents for HPMCAS and/or HPMCP" of the present invention means substances that promote dissolution of the HPMCAS and/or HPMCP, but it does not include substances that promote dissolution of the poorly soluble drug to such an extent that dispersibility of the fine particles of the present invention decreases. This is because if dissolution of the poorly soluble drug itself is promoted during the production of the fine particles of the present invention, aggregation of the fine particles of the present invention occurs and fine particles that are dispersible and have the desired average particle diameter will not be obtained, even if a dispersant such as HPMCAS and/or HPMCP is present. In addition, the HPMCAS and HPMCP that is supposed to have a low solubility must be dissolved and dispersed in order to produce the fine particles of the present invention, but it is not necessarily dissolved to transparency and the solution can be in a turbid state as long as no solid can be seen. The HPMCAS or HPMCP resolvent should be an alkali substance or a substance that will electrolytically dissociate alkali metal ions or alkali earth metal ions in water. Specific examples are sodium citrate, calcium citrate, citrates, sodium tartrate, sodium lactate, and other organic acid salts, sodium hydroxide, potassium hydroxide, calcium hydroxide, magnesium hydroxide, sodium carbonate, sodium bicarbonate, triethanolamine, monoethanolamine, magnesium aluminum silicate, phosphate, magnesium oxide, aqueous ammonia, *L*-arginine, and sodium alginate, and one or a combination of two or more of these substances can be used in the present invention. Moreover, the resolvent for HPMCAS or HPMCP can be an alkaline electrolyte, but as long as it is an electrolyte capable of dispersing the HPMCP or HPMCAS to dissolution or a turbid state, it is not necessarily alkaline and can have a pH near neutrality. It is particularly preferred that pH after dissolution of the HPMCAS or HPMCP is 4 or greater. Ethanol or another water-soluble organic solvent or a mixture of electrolyte and water-soluble organic solvent can be used. However, organic solvents such as ethanol generally show a tendency toward dissolving poorly soluble drugs and there is therefore a tendency toward the dissolved drug concentration in the suspension increasing and the dissolution rate and precipitation rate increasing during the equilibrium process between dissolution and precipitation at the drug particle surface. Consequently, it is possible that aggregation between particles will be promoted during the step when the dissolved drug reprecipitates and the fine particles of the present invention with the desired particle diameter will not be obtained. As a result, it is preferred that the water-soluble organic solvent or mixture of electrolyte and water-soluble organic solvent used in the present invention be used in an amount that does not interfere with the effect of the fine particles of the present invention.

Various pharmaceutical additives can be used as needed to make a pharmaceutical preparation of the fine particles of the present invention. There are no special restrictions to the pharmaceutical additives as long as they are pharmaceutically acceptable additives. Examples of this type of additive are excipients, binders, disintegrating agents, sour flavoring agents, foaming agents, sweeteners, fragrances, lubricants, and coloring agents. Examples of excipients are lactose, crystalline cellulose, microcrystalline cellulose, D-soribitol, and D-mannitol. Examples of binders are hydroxypropylmethyl cellulose, hydroxypropyl cellulose, povidone, polyvinyl alcohol, methyl cellulose, and gum arabic. Examples of disintegrating agents are corn starch, potato starch, carmellose, carmellose calcium, carmellose sodium, croscarmellose sodium, low-substituted hydroxypropyl cellulose, and crospovidone. Examples of sour flavoring agents are citric acid, tartaric acid and malonic acid. An example of a foaming agent is sodium bicarbonate. Examples of sweeteners are saccharine sodium, dipotassium glycyrrhizinate, aspartame, stevia, and somatin. Examples of fragrances are lemon, lemon-lime, orange, and menthol. Examples of lubricants are magnesium stearate, calcium stearate, sucrose fatty acid ester, polyethylene glycol, talc, and stearic acid. Examples of coloring agents are yellow iron sesquioxide, red iron sesquioxide, titanium oxide, yellow food colorings No. 4 and No. 5, red food colorings No. 3 and No. 102, and blue food coloring No. 3. One or a combination of two or more of these pharmaceutical additives can be added in the appropriate amount.

The phrase "wet pulverization" in the present invention means the method whereby the particle size of a drug in a suspension is reduced using a mechanical means or physical phenomenon. Specifically, this method is wet pulverization using a media mill such as a bead or sand mill, high pressure emulsifier, or rotary disk mill. Wet pulverization using a bead mill is preferred. Examples of methods for producing the the present invention are (1) a production method characterized in that a poorly soluble drug is dispersed in a solvent in which HPMCAS and/or HPMCP and a resolvent therefor have been dissolved or suspended, and then the average particle diameter of the poorly soluble drug is reduced by further subjecting the resulting mixture to wet pulverization; (2) a production method characterized in that an organic solvent solution in which a poorly soluble drug has been dissolved is added to a solution in which HPMCAS and/or HPMCP and a resolvent therefor have been dissolved or suspended and fine particles of poorly soluble drug are precipitated; and (3) a production method characterized in that a solvent in which HPMCAS and/or HPMCP and a resolvent therefor have been dissolved or suspended is added to the pulverized product resulting from wet pulverization of a poorly soluble drug in the presence of a dispersant, but production method (1) is preferred.
Furthermore, the enteric base material has an excellent effect in terms of productivity and preventing aggregation during pulverization of the poorly soluble drug with a bead mill, high-pressure emulsifier, or rotary disk mill, but it is also effective as a dispersant for wet pulverization as well as a dispersant for crystallization.

The method for producing the fine particles of the present invention will now be described in detail.
First, the method for production by wet pulverization involves the addition to purified water of HPMCAS and/or HPMCP and a resolvent such as sodium citrate for the dissolution thereof, and agitation to dissolve the HPMCAS and/or HPMCP. Depending on pH, the solution may be transparent, but there are cases in which it is turbid and this is not a problem as long as the enteric base material obviously does not remain in a solid state. Next, the poorly soluble drug is poured into the solution. The average particle diameter of the poorly soluble drug is 500 µm or smaller, preferably 100 µm or smaller, particularly 20 µm or smaller. Moreover, if the average particle diameter of the drug before pulverization exceeds 500 µm, it should be used after being pre-pulverized by dry pulverization with a pin mill to reduce the particle diameter. There is a tendency toward pulverization becoming less difficult as the concentration of the poorly soluble drug in the suspension increases. However, in order to prevent viscosity from rising considerably, the amount of drug added is limited to approximately 30% (w/vol), preferably 1 to 25% (w/vol), particularly 3 to 15%. After the mixed slurry obtained in this way has been set aside overnight and degassed, it is poured into a bead mill, high-pressure emulsifier, rotary disk mill, or other wet pulverizing mill and the device is operated until fine particles with the desired particle diameter are obtained. Moreover, degassing is not always necessary, but pulverization efficiency is improved by degassing. Moreover, defoaming agent, surfactant, and preservative can be added and the solution can be pulverized in order to make the mixed slurry easier to handle and to improve quality.

Beads made from a variety of materials can be used when the particles are pulverized using a bead mill, but beads made of polystyrene, urethane, or another plastic, or of zirconia or another inorganic material are generally used. Pulverization efficiency increases with an increase in bead density, but the bead material is selected taking into consideration the possibility of contamination as a result of abrasion of the beads and the time needed for pulverization. The particle diameter of the beads that are introduced to the container is usually 0.05 mm to 3 mm, preferably 0.1 to 0.5 mm. The amount of beads introduced is preferably 50 to 90%, particularly 70 to 85%, in terms of the percentage filled in the container. Rotors (agitator disks) are turned in order to turn the beads under high speed in the container, and this turning speed is preferably 5 to 12 m/sec, particularly 7 to 11 m/sec, by peripheral speed of the rotors. Moreover, pulverization efficiency is improved when the container is cooled with cooling water.

The mechanism of pulverization by a high-pressure emulsifier is not the grinding mechanism of beads in a bead mill, but rather pulverization under the force of cavitation that is generated when a fluid flows into narrow slits and holes under high pressure and the shear force around the slits. Pulverization occurs as a result of the mixed fluid of poorly soluble drug and enteric base material and a resolvent therefor passing any number of times through these holes and slits, and the desired fine particles are usually obtained when this fluid passes through these slits and holes several times to a few dozens times.

Furthermore, it is also possible to use a rotary disk mill for wet pulverization. A rotary disk mill is a device that applies shear force and pulverizes as a result of a fluid containing the substance to be pulverized passing through the narrow space between a top and bottom disk. The space through which the fluid passes is not necessarily created by round disks and can be conical. A colloid mill and the Clear SS5 (MTechnique, Japan) are cited as typical devices, and modified versions can also be used. By means of the rotary disk mill, a fluid containing poorly soluble drug and hydroxypropylmethyl cellulose acetate succinate and/or hydroxypropylmethyl cellulose phthalate and a resolvent such as sodium citrate for the dissolution thereof is guided from the center of the rotating disk or rotor, and the poorly soluble drug is moved to the periphery and ejected as it is finely crushed under the shear force that is generated by the turning of the disk or rotors. The shear force that is applied to the fluid increases as the space between the disks or rotors becomes narrower, or the number of revolutions of the disks or rotors increases, and the poorly soluble drug can be finely pulverized.

Another method is the method whereby a poorly soluble drug is wet pulverized using PVP, HPMC, or another dispersant and hydroxypropymethyl cellulose acetate succinate and/or hydroxypropylmethyl cellulose phthalate that have been dissolved by a resolvent is added to the resulting suspension and adsorbed.

In addition to the above-mentioned wet pulverization, the fine particles of the present invention can be produced by crystallization.
By means of the crystallization method, HPMCAS and/or HPMCP and a resolvent such as sodium citrate for the dissolution thereof are added to purified water and agitated, and this solution (1) in which the HPMCAS and/or HPMCP have been dissolved is used, as in production by wet pulverization. Depending on pH, the solution (1) may be transparent, but there are cases in which it is turbid and this is not a problem as long as the HPMCAS and/or HPMCP obviously do not remain in a solid state. pH of this solution is adjusted with a phosphate or alkali, depending on the case. There are times when benzalkonium chloride or another preservative is added. On the other hand, a solution of the poorly soluble drug dissolved in ethanol or another organic solvent is used as solution (2). This solution (2) is preferably mixed with the above-mentioned surfactant, which is a dispersant used for both HPMCP and/or HPMCAS in the present invention. Next, solution (1) is agitated as solution (2) of the poorly soluble drug dissolved in ethanol or another organic solvent is slowly being added drop wise. The solution becomes turbid with drop wise addition and fine particles of the poorly soluble drug precipitate. The organic solvent in which the poorly soluble drug is dissolved can be any solvent that will dissolve in water, including acetone, ethanol, methanol, isopropanol, and the like, and it is preferred that the poorly soluble drug be dissolved to a high concentration such that the amount of organic solvent that is used is generally low. The particle diameter of the resulting fine particles is dependent on the agitation conditions and temperature of the poor solvent phase, and the like; therefore, the conditions are set as needed. Moreover, the resulting fine particle suspension contains organic solvent; therefore, the water-dispersible suspension can be produced by removing the solvent phase of the suspension with a filter. It is also possible to remove the solvent phase containing the organic solvent by freeze drying. In addition, drop wise addition of poor solvent phase to the good solvent phase and precipitation of fine particles of poorly soluble drug is possible rather than the vice-versa drop wise addition of poorly soluble drug dissolved in good solvent to poor solvent as in the above-mentioned production method.

Solidification of a fine particle suspension with excellent dispersibility is possible by adding and dissolving or partially dissolving a specific amount of a sugar or sugar alcohol in a poorly soluble drug suspension produced as described above. The drying method can be ventilation drying or vacuum drying, or the surface of round granules of sucrose known by the brand name of Nonpareil can be fluid bed dried as it is being sprayed.
The present invention can be used as an oral agent in order to improve absorption of fine particles of a poorly soluble drug, or the excellent dispersion stability of the suspension can be used for an injection, suspension, syrup, or other liquid, or a semisolid agent. Moreover, the dry product of the fine particle suspension of the present invention can be mixed with other fillers and subjected to various types of formulation treatments to obtain tablets, powder, granules, pills, capsules, sachet, or other solid preparation, and these can be further coated with an enteric film. It is preferred that a sugar and/or sugar alcohol be added in order to maintain redispersibility of the above-mentioned solid preparation that uses the dry fine particle suspension.

The fine particles of poorly soluble drug of the present invention are fine particles that have excellent dispersibility and have an improved dissolution profile as a result of a reduction in particle diameter and increase in surface area. That is, the present invention is technology with which although drug solubility is left low, the drug is made into fine particles and surface area is therefore greatly increased and the amount of drug that is dissolved in the gastrointestinal tract is essentially increased as a result.

### Brief Description of the Drawings

Figure 1 shows the effect of various dispersants on average particle diameter of compound A and the pulverization time in a bead mill.
Figure 2 shows the effect of various dispersants on average particle diameter of compound B and the pulverization time in a bead mill.
Figure 3 shows the effect of various dispersants on average particle diameter of compound C and the pulverization time in a bead mill.

### Preferred Embodiments of the Invention

The present invention will now be described in detail. Working examples and comparative examples are cited to describe the present invention in further detail, but that is in no way to be interpreted as intending the present invention to be limited to these examples.

### [Working Example 1]

As shown in the composition in Table 1, one gram of HPMCP (Shin-Etsu Chemical Co., Ltd., HP-55S) was dissolved in an aqueous solution of sodium citrate dihydrate, pH was adjusted to 6.3 with an aqueous sodium hydroxide solution, five grams of 2E)-3-(4-chlorophenyl)-N-[(1S)-2-oxo-2-[[2-oxo-2-(4-[[6-(trifluoromethyl)-4-pyrimidinyl]oxy]-1-piperidinyl)ethyl] amino]-1-(2-pyridylmethyl)ethyl]-2-propenamide (compound A hereafter), a basic, poorly soluble drug, were dispersed in this liquid, and 100 g of a slurry-like mixture were obtained. Then zirconia beads with a diameter of 0.3 mm (Nikkato Corporation) were packed to a packing density of 80% in the batch-type Dynomill Multilab (WAB, Switzerland), 100 g of this slurry-like mixture were wet pulverized for a pre-determined time at a rotor turning speed of 9 m/sec, and the fine particles of Working Example 1 were obtained.

### [Working Example 2]

The fine particles of Working Example 2 were obtained by the same method as in Working Example 1, with the exception that one gram of HPMCAS (Shin-Etsu Chemical Co., Ltd., AQOAT AS-LG) was used in place of the one gram of HPMCP, as shown in the composition in Table 1.

### [Comparative Example 1]

The fine particles of Comparative Example 1 were obtained by the same method as in Working Example 1, with the exception that one gram of PVP (BASF, PVP K15) was used in place of the one gram of HPMCP, the sodium citrate dihydrate was not added, and pH was not adjusted with sodium hydroxide, as shown in the composition in Table 1.

### [Comparative Example 2]

The fine particles of Comparative Example 2 were obtained by the same method as in Working Example 1, with the exception that five grams of PVP (BASF, PVP K15) were used in place of the one gram of HPMCP, the sodium citrate dihydrate was not added, and pH was not adjusted with sodium hydroxide, as shown in the composition in Table 1.

### [Comparative Example 3]

The fine particles of Comparative Example 3 were obtained by the same method as in Working Example 1, with the exception that one gram of HPMC (Shin-Etsu Chemical Co., Ltd., TC-5R) was used in place of the one gram of HPMCP, the sodium citrate dihydrate was not added, and pH was not adjusted with sodium hydroxide, as shown in the composition in Table 1.

### [Comparative Example 4]

The fine particles of Comparative Example 4 were obtained by the same method as in Working Example 1, with the exception that 2.5 g of HPMC (Shine-Etsu Chemical Co., Ltd., TC-5R) were used in place of the one gram of HPMCP, the sodium citrate dihydrate was not added, and pH was not adjusted with sodium hydroxide, as shown in the composition in Table 1.

### <Test Example 1: Relationship between pulverization time and average particle diameter with basic, poorly soluble drug compound A>

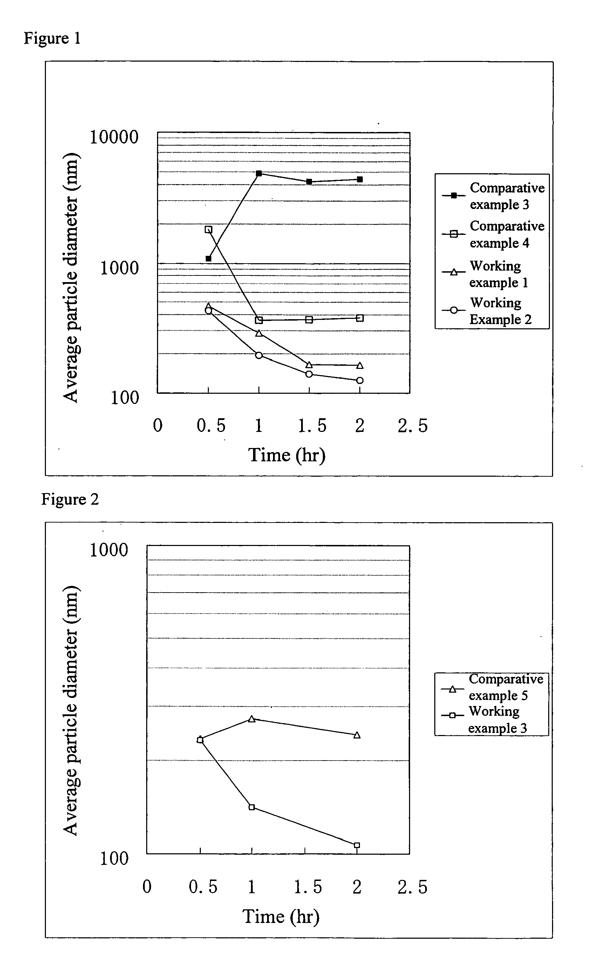

The average particle diameter of the suspension fine particles of Working Examples 1 and 2 and Comparative Examples 3 and 4 obtained by wet pulverization for a predetermined time were measured using the Horiba LA-920 (Horiba Ltd.), a particle diameter measuring device based on laser scattering. The correlation between pulverization time and average particle diameter is shown in Figure 1. A suspension of primary particles of 10 µm or smaller was not obtained by pulverization in Comparative Examples 1 and 2, which used PVP. Moreover, the average particle diameter with pulverization for two hours was 377.3 nm in Comparative Example 3, which used HPMC, but it reached 150 nm in Working Examples 1 and 2, which used HPMCAS or HPMCP, and it was concluded that the pulverization time is curtailed considerably and that the particle diameter that is realized after pulverization is smaller when the HPMCAS or HPMCP of the present invention is used. Curtailing the pulverization time in this way is particularly useful for efficient production and production control on an actual production scale. It appears that the HPMCAS and HPMCP used in the present invention not only are effective in the fine pulverization of a poorly soluble drug in a short amount of time, but also participate in the improvement of dispersibility over time, which is discussed later.

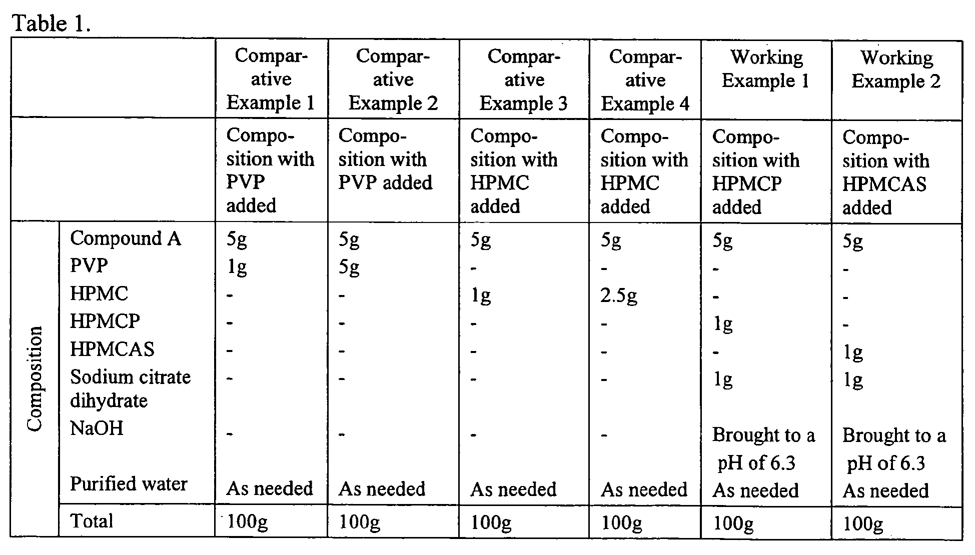

### [Working Example 3]

The fine particles of Working Example 3 were obtained as in Working Example 2, with the exception that five grams of the basic, poorly soluble drug (2E)-3-[4-(1H-benzimidazol-2-ylmethyl)phenyl]-*N-*hydroxyacrylamide (abbreviated as Compound B hereafter) were used in place of the five grams of basic, poorly soluble compound A, as shown in Table 2.

### [Comparative Example 5]

The fine particles of Comparative Example 5 were obtained as in Comparative Example 1, with the exception that five grams of basic, poorly soluble compound B were used in place of the five grams of basic, poorly soluble compound A, as shown in Table 2.

### <Test Example 2: Relationship between pulverization time and average particle diameter with basic, poorly soluble drug compound B>

The average particle diameter of the suspension fine particles of Working Example 3 and Comparative Example 5 obtained by wet pulverization for a predetermined time were measured using the Horiba LA-920, a particle diameter measuring device based on laser scattering. The correlation between pulverization time and average particle diameter is shown in Figure 2. A suspension of fine particles having an average particle diameter of 200 nm or smaller was not obtained in Comparative Example 5, which used PVP, even with pulverization for two hours, but in the case of Working Example 3 (AQOAT AS-LG), which used HPMCAS, a suspension of fine particles of 200 nm or smaller was obtained in one hour, and a particle diameter of 106.5 nm was attained in two hours.

### [Working Example 4]

The fine particles of Working Example 4 were obtained as in Working Example 2, with the exception that five grams of poorly soluble drug 3-methoxy-1.5-bis(4-methoxyphenyl)-1H-1,2,4-triazole (abbreviated as compound C hereafter) were used in place of the five grams of basic, poorly soluble drug compound A, as shown in Table 2.

### [Comparative Example 6]

The fine particles of Comparative Example 6 were obtained as in Comparative Example 1, with the exception that five grams of poorly soluble compound C were used in place of the five grams of basic, poorly soluble drug compound A, as shown in Table 2.

### [Comparative Example 7]

The fine particles of Comparative Example 7 were obtained as in Comparative Example 3, with the exception that five grams of poorly soluble drug compound C were used in place of the five grams of basic, poorly soluble drug compound A, as shown in Table 2.

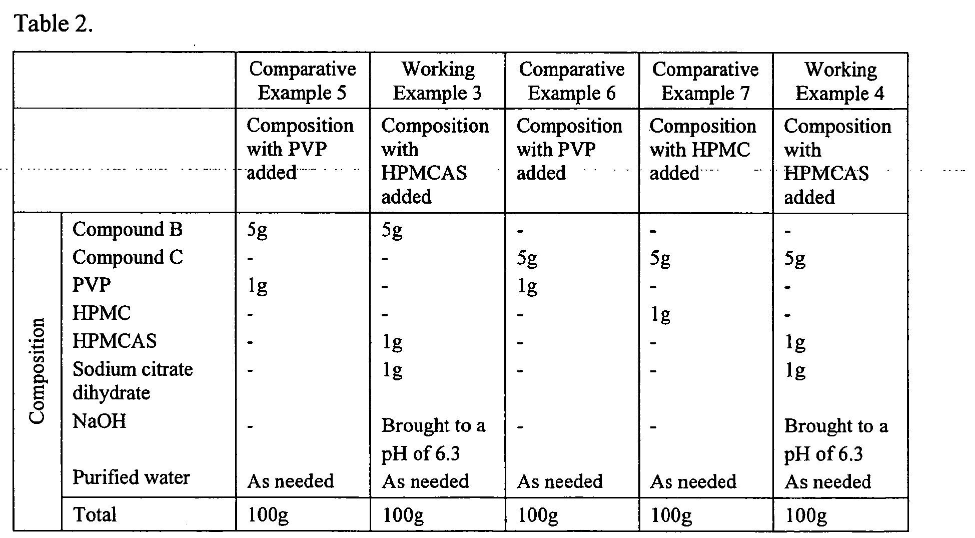

### <Test Example 3: Relationship between pulverization time and average particle diameter with poorly soluble drug compound C>

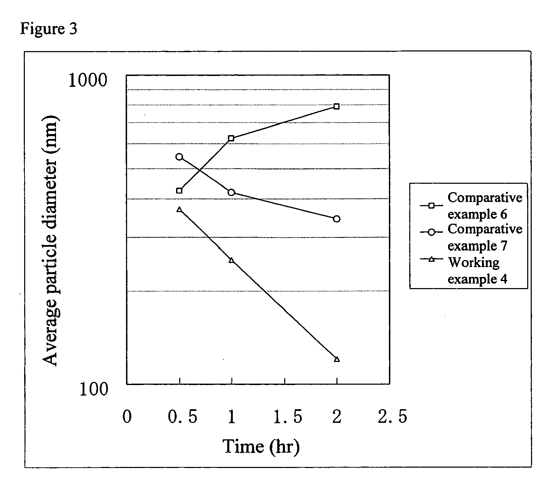

The average particle diameter of the suspension fine particles of Working Example 4 and Comparative Examples 6 and 7 obtained by wet pulverization for a predetermined time were measured using the Horiba LA-920, a particle diameter measuring device based on laser scattering. The correlation between pulverization time and average particle diameter is shown in Figure 3. A suspension of fine particles of 400 nm or smaller was not obtained by pulverization in Comparative Example 6, which used PVP. In addition, in contrast to the fact that the average particle diameter with pulverization for two hours was 343 nm in Comparative Example 7, which used HPMC, an average particle diameter of 120.4 nm was reached with Working Example 4, which used HPMCAS.

### <Test Example 4: Dilution stability of suspension in dissolution testing fluid No. 2 of Japanese Pharmacopoeia>

Eleven milliliters of dissolution testing fluid No. 2 of the Japanese Pharmacopoeia (pH of 6.8) were introduced to the measurement cell of the Horiba LA-920 (Horiba Ltd.), a particle diameter measuring device based on laser scattering; the blank scattering light was stored; the suspensions produced by Working Examples 1 and 2 and Comparative Example 4 were added with an Eppendorf pipette up to a dilution rate of 730-times; and the particle diameter was measured over time. Table 3 shows the average particle diameter of each suspension. The average particle diameter of Comparative Example 3 which used HPMC, was 377.3 nm, the particles aggregated to a diameter of 12,549.7 nm after 60 minutes when the suspension was diluted with dissolution test fluid No. 2 of the Japanese Pharmacopoeia. On the other hand, there was no change in the particle diameter of Working Examples 1 and 2, which used HPMCAS and HPMCP.
It was clear that drug particles with a small particle diameter are obtained in a short time in Working Examples 1 and 2, which used HPMCAS or HPMCP, and that dispersion stability over time is also excellent.

**Table 3**

| | | Average particle diameter after dilution 730-times with fluid No.2 | | |
|---|---|---|---|---|
| | | Before dilution | 30 minutes after dilution | 60 minutes after dilution |
| Comparative Example 4 | Composition with HPMC added | 377.3 nm | - | 12549.7 nm |
| Working Example 1 | Composition with HPMCP added | 164.2 nm | 174.3 nm | 176.2 nm |
| Working Example 2 | Composition with HPMCAS added | 125.5 nm | 138.3 nm | 131.9 nm |

### <Test Example 5: Redispersibility of dry fine particle suspension>

Fifty milligrams of lactose were added and dissolved in two grams of the fine particle suspension produced by Working Example 1 and the mixture were placed in a Teflon sheet tray and ventilation dried at 40°C. The dry product was pulverized and sifted with a 50 µm sieve to obtain a fine particle suspension dry sample. A dry sample to which lactose was not added was also prepared. Ten milligrams of these samples were introduced to a test tube, 0.5 mL of purified water or fluid No. 2 was added, the samples were redispersed with a lab mixer, and the average particle diameter was measured. The results are shown in Table 4. The dry fine particle suspension to which lactose had been added displayed superior redispersibility.

**Table 4.**

| Presence of lactose | Average particle diameter before drying | Average particle diameter after drying | |
|---|---|---|---|
| | | After redispersion in purified water | After redispersion in fluid No. 2 |
| Lactose-free | 163.2 nm | 303.8 nm | 15448.9 nm |
| Lactose added | 164.7 nm | 170.7 nm | 179.7 nm |

### [Working Example 5]

One gram of HPMCAS (Shin-Etsu Chemical Co., Ltd., AQOAT AL-LG) was dissolved in an aqueous solution of sodium citrate dihydrate, pH was brought to 6.3 with an aqueous sodium hydroxide solution, and five grams of basic, poorly soluble drug compound A and 0.02 g of sodium laurylsulfate were dispersed in this solution to obtain 100 g of a slurry-like mixture. Then zirconia beads with a diameter of 0.3 mm (Nikkato Corporation) were packed in the batch-type Dynomill Multilab (WAB, Switzerland) to a packing density of 80%, and 100 g of this slurry-like mixture were wet pulverized for two hours at a rotor turning speed of 9 m/sec to obtain fine particles with an average particle diameter of 131 nm.

### [Working Example 6]

As shown in the composition in Table 5, HPMCAS (Shin-Etsu Chemical Co., Ltd., AQOAT AS-LG) was dissolved in an aqueous sodium citrate dihydrate solution, pH was adjusted to 6.3 with sodium hydroxide to obtain solution 1, this solution 1 was agitated by 6,000 turns using a homogenizer, solution 2 of the basic poorly soluble drug compound A and sodium laurylsulfate dissolved in ethanol was added, and fine particles with an average particle sum of 356.4 nm were precipitated.

**Table 5**

| | | Working Example 6 |
|---|---|---|
| Solution 1 | HPMCAS | 5 g |
| | Sodium citrate dihydrate | 5 g |
| | NaOH | As needed (to adjust pH to 6.3) |
| | Purified water | As needed |
| | Total | 1,000 g |
| Solution 2 | Compound A | 10 g |
| | Sodium laurylsulfate | 0.5 g |
| | Ethanol | As needed |
| | Total | 40 g |

### [Preparation of Suspension 1]

As shown in Table 6, 15 g of the basic, poorly soluble drug compound A and 0.1 g of sodium laurylsulfate were dispersed in a solution of 3 g of HPMC dissolved in water to obtain 100 g of a slurry mixture. Then zirconia beads with a diameter of 0.3 mm (Nikkato Corporation) were packed in the batch-type Dynomill Multilab (WAB, Switzerland) to a packing density of 80%, and 100 g of this slurry mixture were wet pulverized for two hours at a rotor turning speed of 9 m/sec to obtain fine particles with an average particle diameter of 154 nm (suspension 1).

**Table 6**

| | | Suspension 1 |
|---|---|---|
| Composition | Compound A | 15 g |
| | HPMC | 3 g |
| | Sodium laurylsulfate | 0.15 g |
| | Purified water | As needed |
| | Total | 100 g |

### [Working Example 7]

As shown in Table 7, Working Example 7 was obtained by mixing 20 g of suspension 1 and HPMCP (Shin-Etsu Chemical Co., Ltd., HP-55S) that had been dissolved using an aqueous sodium citrate dihydrate solution and brought to a pH of 6.3 using sodium hydroxide.

### [Working Example 8]

As shown in Table 7, Working Example 8 was obtained by the same method as Working Example 7, with the exception that 0.6 g of HPMCAS (Shin-Etsu Chemical Co., Ltd., AQOAT AS-LG) was used in place of the 0.6 g of HPMCP.

### [Comparative Example 8]

As shown in Table 7, Comparative Example 8 was obtained by the same method as Working Example 7, with the exception that 0.6 g of HPIVIC was used in place of the 0.6 g of HPMCP, sodium citrate dihydrate was not added, and pH was not adjusted using sodium hydroxide.

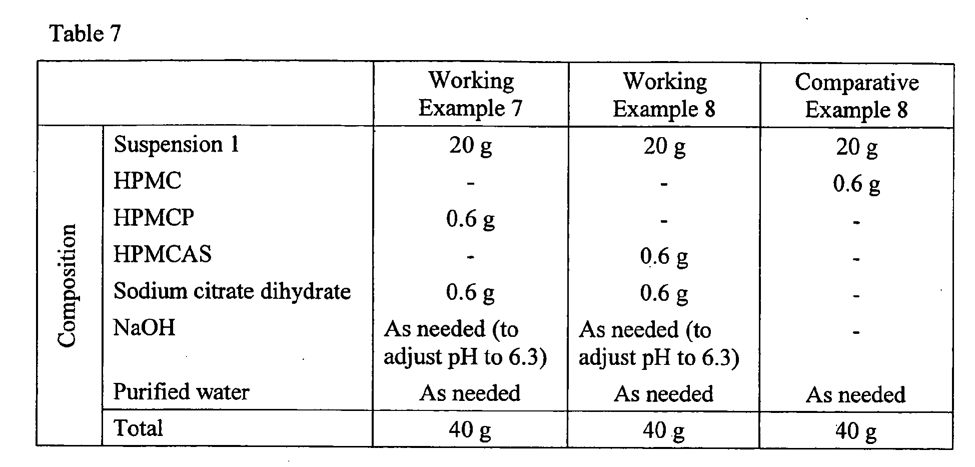

### <Test Example 6: Dilution stability of suspension 1 containing HPMCP or HPMCAS in dissolution testing fluid No. 2 of the Japanese Pharmacopoeia>

100 mL of dissolution testing fluid No. 2 of the Japanese Pharmacopoeia were added to 0.1 mL of the suspension of Working Example 7, Working Example 8, and Comparative Example 8 to obtain a 1:1,000 dilution, and particle diameter over time was measured.
Table 8 shows the average particle diameter of each suspension. When the suspension of Comparative Example 8, which used HPMC alone, was diluted with dissolution testing fluid No. 2 of the Japanese Pharmacopoeia, the particles aggregated to a size of 3,034.2 nm after 60 minutes. On the other hand, there were virtually no changes in particle diameter beginning immediately after dilution with dissolution testing fluid No. 2 of the Japanese Pharmacopoiea in Working Example 7 or 8 to which HPMCP or HPMCAS was added after preparation of the suspension.
It became clear that dispersion stability over time is improved by adding HPMCP or HPMCAS dissolved in an alkali after preparing an HPMC suspension, which tends to aggregate when diluted with tesing fluid No. 2 of the Japanese Pharmacopoeia.

**Table 8**

| | Average particle diameter after 1:1,000 dilution with fluid No. 2 | | | | |
|---|---|---|---|---|---|
| | Before dilution | Immediately after dilution | 30 minutes after | 60 minutes after | 180 minutes after |
| Working Example 7 (HPMCP added) | 153.7 nm | 246.0 nm | 251.2 nm | 261.6 nm | 241.4 nm |
| Working Example 8 (HPMCAS added) | 128.2 nm | 290.1 nm | 288.6 nm | 271.2 nm | 288.7 nm |
| Comparative Example 8 (HPMC added) | - | 273.7 nm | 1,386.4 nm | 3,034.2 nm | 2,860.8 nm |

### Industrial Applicability

The present invention relates to fine particles of a poorly soluble drug wherein a predetermined enteric base material has been adsorbed on the surface of a poorly soluble drug; fine particles further containing a sugar; and a method for producing the same. By using the present invention, it is possible to efficiently and safely produce in a short amount of time fine particles with which absorption of a poorly soluble drug that is poorly absorbed in humans, and the like can be improved, and a pharmaceutical preparation with excellent dispersion stability can be provided.

## Claims

1. Fine particles of a poorly soluble drug, having an average particle diameter of 1 nm to 1,000 nm, wherein hydroxypropylmethyl cellulose acetate succinate and/or hydroxypropylmethyl cellulose phthalate are adsorbed on the surface of the poorly soluble drug.

2. The fine particles according to claim 1, which comprise hydroxypropylmethyl cellulose acetate succinate and/or hydroxypropylmethyl cellulose phthalate at a ratio of 0.005 to 20 parts by weight per part by weight of the poorly soluble drug.

3. The fine particles according to either of claims 1 and 2, which further comprise 0.01 to 4,000 parts by weight of a sugar and/or a sugar alcohol per part by weight of the poorly soluble drug.

4. The fine particles according to claim 3, wherein the sugar and/or the sugar alcohol is one or more selected from the group consisting of lactose, fructose, sucrose, glucose, oligosaccharides, mannitol, sorbitol, maltitol, maltose, xylitol, erythritol, reduced thick maltose syrup, trehalose, anhydrous lactose, and xylose.

5. The fine particles according to any of claims 1 through 4, which are obtainable by a production method selected from the following (1) through (3):
(1) a production method **characterized in that** a poorly soluble drug is dispersed in a solvent in which hydroxypropylmethyl cellulose acetate succinate and/or hydroxypropylmethyl cellulose phthalate and a resolvent thereof have been dissolved or suspended, and then the average particle diameter of the poorly soluble drug is reduced by further subjecting the resulting mixture to wet pulverization;
(2) a production method **characterized in that** an organic solvent solution in which a poorly soluble drug has been dissolved is added to a solution in which hydroxypropylmethyl cellulose acetate succinate and/or hydroxypropylmethyl cellulose phthalate and a resolvent thereof have been dissolved or suspended and fine particles of a poorly soluble drug are precipitated; or
(3) a production method **characterized in that** a solvent in which hydroxypropylmethyl cellulose acetate succinate and/or hydroxypropylmethyl cellulose phthalate and a resolvent therefor have been dissolved or suspended is added to the pulverized product resulting from wet pulverization of a poorly soluble drug in the presence of a dispersant.

6. The fine particles according to claim 5, wherein the resolvent for hydroxypropylmethyl cellulose acetate succinate and/or hydroxypropylmethyl cellulose phthalate is an alkaline substance or a substance that electrolytically dissociates alklali metal ions or alkali earth metal ions in water.

7. The fine particles according to claims 5 and 6, wherein the alkaline substance or the substance that electrolytically dissociates alkali metal ions or alkali earth metal ions in water is one or more substances selected from the group consisting of sodium citrate, calcium citrate, citrates, sodium tartrate, sodium malate, sodium lactate, sodium hydroxide, potassium hydroxide, calcium hydroxide, magnesium hydroxide, sodium carbonate, sodium bicarbonate, triethanolamine, monoethanolamine, magnesium aluminum silicate, phosphate, magnesium oxide, calcium oxide, *L-*arginine, aqueous ammonia, and sodium alginate.

8. A method for producing fine particles of poorly soluble drug having an average particle diameter of 1 nm to 1,000 nm, wherein hydroxypropylmethyl cellulose acetate succinate and/or hydroxypropylmethyl cellulose phthalate are adsorbed on the surface of the poorly soluble drug, **characterized in that** solublehydroxypropylmethyl cellulose acetate succinate and/or hydroxypropylmethyl cellulose phthalate and a resolvent therefor are dissolved or suspended in water or a mixture of a water-soluble organic solvent and water, a poorly soluble drug is dispersed therein, and then the average particle diameter of the poorly soluble drug is reduced by further subjecting the resulting mixture to wet pulverization.

9. A method for producing fine particles of poorly soluble drug, having an average particle diameter of 1 nm to 1,000 nm, wherein hydroxypropylmethyl cellulose acetate succinate and/or hydroxypropylmethylcellulose phthalate are adsorbed on the surface of the poorly soluble drug, **characterized in that** an organic solvent solution in which the poorly soluble drug has been dissolved is added to a solution in which hydroxypropylmethyl cellulose acetate succinate and/or hydroxypropylmethyl cellulose phthalate and a resolvent therefor have been dissolved or suspended, and fine particles of poorly soluble drug are precipitated.

10. A method for fine particles of poorly soluble drug, having an average particle diameter of 1 nm to 1,000 nm, wherein hydroxypropylmethyl cellulose acetate succinate and/or hydroxypropylmethyl cellulose phthalate are adsorbed on the surface of the poorly soluble drug, **characterized in that** a solvent in which hydroxypropylmethyl cellulose acetate succinate and/or hydroxypropylmethyl cellulose phthalate and a resolvent therefor have been dissolved or suspended is added to the pulverized product obtainable by wet pulverization of a poorly soluble drug in the presence of a dispersant.
